# EUROPEAN PATENT APPLICATION

(11) **EP 1 541 154 A1**
(43) Date of publication of application: **15.06.2005**
(21) Application number: 03764145.3
(22) Date of filing: 09.07.2003
(51) Int. Cl.: A61K 31/7068, A61P 25/00, C07H 19/10

(54) **MEDICINAL COMPOSITION FOR DRUG-INDUCED NEUROPATHY**

(30) Priority: 11.07.2002 JP 2002202715
(71) Applicant: YAMASA CORPORATION, Choshi-shi, Chiba 288-0056 (JP)
(72) Inventor: ENDO, Kazuki, Narita-shi, Chiba 286-0014 (JP); KAMEI, Junzo, Yokohama-shi, Kanagawa 236-0012 (JP)
(74) Representative: von Kreisler, Alek, Dipl.-Chem.
(86) International application number: PCT/JP2003/008708
(87) International publication number: WO 2004/006940

(57) **Abstract**

The present invention relates to an agent for the prophylaxis or treatment of drug-induced neuropathy, which contains cytidine 5'-diphosphocholine (CDP-choline) as an active ingredient. The agent for the prophylaxis or treatment of drug-induced neuropathy of the present invention shows a potent ameliorating effect on drug-induced neuropathy and is also superior in safety.

## Description

### Technical Field

The present invention relates to a pharmaceutical agent for the prophylaxis or treatment of drug-induced neuropathy, which contains cytidine 5'-diphosphocholine (CDP-choline) as an active ingredient, a method for the prophylaxis or treatment of said disorder, which comprises administering an effective amount of CDP-choline, use of CDP-choline for the production of a pharmaceutical agent for the prophylaxis or treatment of said disorder, and a pharmaceutical composition for the prophylaxis or treatment of said disorder, which comprises an effective amount of CDP-choline and a pharmaceutically acceptable carrier.

### Background Art

At present, it is known that neuropathy such as peripheral neuropathy and the like is induced as side effects of various pharmaceutical products. Peripheral nerve includes sensory nerve, motor nerve and autonomic nerve. If it would be disorderd, various symptoms would be shown depending on disoedered region, remarkably impairing the quality of life of patients. Therefore, sufficient attention has been paid to the use of pharmaceutical products.

As a main pharmaceutical product that causes peripheral neuropathy, antidepressants (imipramine, amitriptyline), antiepileptic agents (phenytoin, carbamazepine, barbitals), antituberculosis agents (isoniazid, ethambutol), antibiotics (chloramphenicol, thiamphenicol), anti-malignant tumor agents (cisplatin, vinca alkaloids (vincristine etc.), procarbazine, paclitaxel, cytarabine), therapeutic agents for AIDS including Highly Active Anti-Retroviral Therapy (HAART) for AIDS (Zidovudine, Lamivudine, Stavudine, Zalcitabine, Didanosine, Abacavir), antirheumatic agents (sodium aurothiomalete), antiarrhythmic agents (amminodarone, dermatological agents used for Reye syndrome (dapsone), antialcoholic agents against excessive alcohol drinking (disulfiram), antihypertensive agents (hydralazine), radiation sensitizers (misonidazole), vitamins (pyridoxine), immunosuppressants (tacrolimus etc.) and the like are known.

Besides these pharmaceutical products, poisonoum substances such as acrylamide, allyl chloride, arsenic, diphteria toxin, hexacarbones, inorganic lead, cadmium, trichloroethylene, organic phosphoric acid and ester thereof, thallium (rodenticide) and the like, and ethanol (alcohol drinking etc.) have been reported to cause peripheral neuropathy. The "drug " in the present specification embraces pharmaceutical products, poisonoum substances and ethanol.

To deal with such drug-induced neuropathy, it is considered most important to stop use, contact or intake of a drug that induces peripheral neuropathy as soon as possible.

Even if use, contact or intake of a drug is stopped, peripheral neuropathy sustains subjective symptoms of paresthesia such as pain, numbness and the like over a long period of time. In addition, suspension of a pharmaceutical product means interruption of the treatment of the primary disease of the patients and thus the development of more desirable method of dealing with drug-induced neuropathy has been desired.

As a method of dealing with drug-induced neuropathy, for example, it is known that the onset of peripheral neuropathy induced by isoniazid which is an antituberculosis agent, can be prevented by the concurrent use of vitamin B6. In addition, it has been reported that triacetyluridine is effective for hyposensitivity, which is one symptom of peripheral neuropathy induced by Taxol, which is an anticancer agent [WO 00/11952].

However, vitamin B6 is not used for neuropathy induced by a drug other than isoniazid, and there is no drug that shows an almighty effect on neuropathy induced by a drug.

In addition, the above international publication does not consider or even suggest whether or not triacetyluridine is effective for neuropathy induced by a drug other than Taxol. Moreover, the above international publication exemplarily indicates CDP-choline, other than triacetyluridine, as a nucleic acid-related compound. However, CDP-choline is merely shown to have such possibility therein, without any data verifying the effectiveness of CDP-choline on peripheral neuropathy.

### Disclosure of the Invention

In view of the above situation, the present inventors have intensively studied the efficacy of nucleic acid related compounds on drug-induced neuropathy and found that, while having been conventionally permitted for use only by intravenous administration for the application to disorders in the central nerve system, such as consciousness disorder associated with head injury and brain operation, CDP-choline surprisingly shows, even by oral administration, a superior improving effect on drug-induced neuropathy such as hyperesthesia and the like, which resulted in the completion of the present invention.

Accordingly, the present invention provides the following.
[1] An agent for the prophylaxis or treatment of drug-induced neuropathy, which comprises cytidine 5'-diphosphocholine or a pharmaceutically acceptable salt thereof as an active ingredient.
[2] The agent of the above-mentioned [1], wherein the drug-induced neuropathy is peripheral neuropathy.
[3] The agent of the above-mentioned [1], which is in a dosage form for oral administration.
[4] The agent of the above-mentioned [1], wherein the drug-induced neuropathy is induced by a pharmaceutical product selected from the group consisting of antidepressant, antiepileptic agent, antituberculosis agent, antibiotics, anti-malignant tumor agent, a therapeutic agent for AIDS, antirheumatic agent, antiarrhythmic agent, dermatological agent used for Reye syndrome, antialcoholic agent against excessive alcohol drinking, antihypertensive agent, radiation sensitizer, vitamin and immunosuppressant.
[5] The agent of the above-mentioned [1], wherein the drug-induced neuropathy is induced by poisonoum substance or ethanol.
[6] A method for the prophylaxis or treatment of drug-induced neuropathy, which comprises administering an effective amount of cytidine 5'-diphosphocholine or a pharmaceutically acceptable salt thereof to a patient.
[7] The method of the above-mentioned [6], wherein the drug-induced neuropathy is peripheral neuropathy.
[8] The method of the above-mentioned [6], wherein the administration comprises oral administration.
[9] The method of the above-mentioned [6], wherein the drug-induced neuropathy is induced by a pharmaceutical product selected from the group consisting of antidepressant, antiepileptic agent, antituberculosis agent, antibiotics, anti-malignant tumor agent, a therapeutic agent for AIDS, antirheumatic agent, antiarrhythmic agent, dermatological agent used for Reye syndrome, antialcoholic agent against excessive alcohol drinking, antihypertensive agent, radiation sensitizer, vitamin and immunosuppressant.
[10] The method of the above-mentioned [6], wherein the drug-induced neuropathy is neuropathy induced by poisonoum substance or ethanol.
[11] Use of cytidine 5'-diphosphocholine or a pharmaceutically acceptable salt thereof for the production of an agent for the prophylaxis or treatment of drug-induced neuropathy.
[12] Use of the above-mentioned [11], wherein the drug-induced neuropathy is peripheral neuropathy.
[13] Use of the above-mentioned [11], wherein the agent is in a dosage form for oral administration.
[14] Use of the above-mentioned [11], wherein the drug-induced neuropathy is neuropathy induced by a pharmaceutical product selected from the group consisting of antidepressant, antiepileptic agent, antituberculosis agent, antibiotics, antimalignant tumor agent, a therapeutic agent for AIDS, antirheumatic agent, antiarrhythmic agent, dermatological agent used for Reye syndrome, antialcoholic agent against excessive alcohol drinking, antihypertensive agent, radiation sensitizer, vitamin and immunosuppressant.
[15] Use of the above-mentioned [11], wherein the drug-induced neuropathy is neuropathy induced by a poisonoum substance or ethanol.
[16] A pharmaceutical composition for the prophylaxis or treatment of drug-induced neuropathy, which comprises an effective amount of cytidine 5'-diphosphocholine or a pharmaceutically acceptable salt thereof, and a pharmaceutically acceptable carrier.
[17] The pharmaceutical composition of the above-mentioned [16], wherein the drug-induced neuropathy is peripheral neuropathy.
[18] The pharmaceutical composition of the above-mentioned [16], which is in a dosage form for oral administration.
[19] The pharmaceutical composition of the above-mentioned [16], wherein the drug-induced neuropathy is neuropathy induced by a pharmaceutical product selected from the group consisting of antidepressant, antiepileptic agent, antituberculosis agent, antibiotics, anti-malignant tumor agent, a therapeutic agent for AIDS, antirheumatic agent, antiarrhythmic agent, dermatological agent used for Reye syndrome, antialcoholic agent against excessive alcohol drinking, antihypertensive agent, radiation sensitizer, vitamin and immunosuppressant.
[20] The pharmaceutical composition of the above-mentioned [16], wherein the drug-induced neuropathy is neuropathy induced by a poisonoum substance or ethanol.
[21] A commercial package comprising the pharmaceutical composition of the above-mentioned [16] and a written matter associated therewith, the written matter stating that the pharmaceutical composition can or should be used for the prophylaxis or treatment of drug-induced neuropathy.

### Brief Description of the Drawings

Fig. 1 shows the results of Tail Flick Test (effect of CDP-choline regarding vincristine-induced neuropathy), wherein the vertical axis shows reaction latency (sec).
Fig. 2 shows the results of Tail Flick Test (effect of CDP-choline regarding acrylamide-induced neuropathy), wherein the vertical axis shows reaction latency (sec).

### Detailed Description of the Invention

The present invention provides an agent for the prophylaxis or treatment of drug-induced neuropathy, which comprises CDP-choline or a pharmaceutically acceptable salt thereof as an active ingredient.

The present invention also provides a method for the prophylaxis or treatment of drug-induced neuropathy, which comprises administering an effective amount of CDP-choline or a pharmaceutically acceptable salt thereof.

The present invention further provides use of CDP-choline or a pharmaceutically acceptable salt thereof for the production of a pharmaceutical agent for the prophylaxis or treatment of drug-induced neuropathy.

The present invention also provides pharmaceutical composition for the prophylaxis or treatment of drug-induced neuropathy, which comprises an effective amount of CDP-choline or a pharmaceutically acceptable salt thereof, and a pharmaceutically acceptable carrier.

The present invention moreover provides a pharmaceutical composition for the prophylaxis or treatment of drug-induced neuropathy, which comprises an effective amount of CDP-choline or a pharmaceutically acceptable salt thereof, and a pharmaceutically acceptable carrier, and a commercial package comprising said pharmaceutical composition and a written matter relating to the pharmaceutical composition, the written matter stating that the pharmaceutical composition can or should be used for the prophylaxis or treatment of drug-induced neuropathy.

CDP-choline as an active ingredient in the present invention may be in a free form or a salt form. Examples of the salt form include, but not limited to, alkali metal salts such as lithium salt, sodium salt, potassium salt and the like; alkaline earth metal salts such as magnesium salt and the like; and the like. Of these, a pharmaceutically acceptable salt is particularly preferable.

CDP-choline or a salt thereof may be a hydrate or a solvate. In the case of a hydrate, a hydrate or a salt hydrate wherein 1 - 20 molecules of water is(are) attached or bonded to 1 molecule of CDP-choline or a salt thereof, is exemplified.

Furthermore, CDP-choline or a salt thereof, or a hydrate or a salt hydrate thereof in the form of a crystal or a noncrystal can be used in the present invention.

Such CDP-choline or a salt thereof (hereinafter to be simply referred to as "CDP-choline") is commercially available as a known compound, and can be produced according to a known method (see, JP-B-6-31306, EP Patent No. 329627, US Patent No. 6057301 and the like).

The pharmaceutical composition of the present invention can be prepared by mixing the aforementioned CDP-choline and a conventional pharmaceutically acceptable carrier (pharmaceutical carrier) and processing the mixture into a pharmaceutical product. The content of CDP-choline in the composition may be appropriately selected from the range of not less than 0.01% (w/w), preferably 1 - 80% (w/w).

As a carrier for the preparation, a substance conventionally used in the field of pharmaceutical preparation, which does not react with CDP-choline, is used. Specific examples include lactose, glucose, mannitol, dextrin, starch, sucrose, magnesium aluminometasilicate, synthetic aluminum silicate, crystalline cellulose, carboxymethylcellulose sodium, hydroxypropyl starch, carboxymethylcellulose calcium, ion exchange resin, methylcellulose, gelatin, gum acacia, hydroxypropylcellulose, low-substituted hydroxypropylcellulose, hydroxypropylmethylcellulose, polyvinylpyrrolidone, polyvinyl alcohol, light anhydrous silicic acid, magnesium stearate, talc, carboxy vinyl polymer, titanium oxide, sorbitan fatty acid ester, sodium lauryl sulfate, glycerine, fatty acid glycerine ester, purified lanolin, glycerinated gelatin, polysorbate, macrogol, vegetable oil, wax, liquid paraffin, white petrolatum, nonionic surfactant, propylene glycol, water and the like.

The dosage form is exemplified by tablet, capsule, granule, powder, syrup, suspension, suppository, ointment, gel, patch, injection, eye drop and the like. These pharmaceutical products can be prepared according to a conventional method appropriately using the above pharmaceutical carrier. A dosage form for oral administration is preferable.

In the case of a pharmaceutical preparation in the form of liquid, it may be in the form that dissolve or suspend in water or other suitable medium when in use. It is also possible to apply coating to tablet and granule by a well-known method.

The target disease of the prophylactic or therapeutic agent or the pharmaceutical composition of the present invention is drug-induced neuropathy, namely, neuropathy induced by the administration, contact or intake of a drug, which is not particularly limited as long as administration of CDP-choline improves symptoms thereof. Specifically, peripheral neuropathy induced by administration of one or more pharmaceutical products such as antidepressants (imipramine, amitriptyline and the like), antiepileptic agents (phenytoin, carbamazepine, barbitals and the like), antituberculosis agents (isoniazid, ethambutol and the like), antibiotics (chloramphenicol, thiamphenicol and the like), anti-malignant tumor agents (cisplatin, vinca alkaloids (vincristine and the like), procarbazine, paclitaxel, cytarabine and the like), therapeutic agents for AIDS including Highly Active Anti-Retroviral Therapy (HAART) of AIDS (Zidovudine, Lamivudine, Stavudine, Zalcitabine, Didanosine, Abacavir and the like), antirheumatic agents (sodium aurothiomalete and the like), antiarrhythmic agents (amminodarone and the like), dermatological agents used for Reye syndrome (dapsone and the like), antialcoholic agents against excessive alcohol drinking (disulfiram and the like), antihypertensive agents (hydralazine and the like), radiation sensitizers (misonidazole and the like), vitamins (pyridoxine and the like), immunosuppressants (tacrolimus and the like) and the like, or peripheral neuropathy induced by contact or intake of one or more poisonoum substances (acrylamide, allyl chloride, arsenic, diphteria toxin, hexacarbones, inorganic lead, cadmium, trichloroethylene, organic phosphoric acid or ester thereof, thallium (rodenticide) and the like) or ethanol can be mentioned. Peripheral neuropathy includes, for example, mononeuropathy caused by an affected single nerve, multiple mononeuropathy caused by two or more affected nerves in different regions, multiple neuropathy caused by many simultaneously affected nerves and the like.

The prophylactic or therapeutic agent or pharmaceutical composition of the present invention are capable of ameliorating particularly the symptoms of peripheral neuropathy (burning sensation, acute pain, numbness, hyperesthesia, hyposensitivity, loss of muscle strength, muscular atrophy, loss of deep tendon reflex and the like), and effective for the prophylaxis or treatment of drug-induced peripheral neuropathy.

The administration method of the agent or pharmaceutical composition for the prophylaxis or treatment of drug-induced neuropathy of the present invention may be any and is selected from oral administration, parenteral administration, intrarectal administration and local administration, depending on the dosage form thereof. Oral administration is preferable.

While the dose of CDP-choline, which is an active ingredient in the present invention, varies depending on the administration method, symptom and age of patient, and the like, it is generally about 0.1 - 1000 mg/kg/day, preferably about 0.5 - 500 mg/kg/day, which can be administered in a single dose or divided doses.

The "prophylaxis of drug-induced neuropathy" in the present invention includes administration of CDP-choline in advance to patients who experienced administration of, contact with or intake of the above-mentioned drug capable of inducing neuropathy but has not developed symptoms of neuropathy or patients scheduled for administration of the above-mentioned pharmaceutical product. For example, CDP-choline is administered simultaneously with the above-mentioned pharmaceutical product capable of inducing neuropathy or before administration, contact with or intake of the above-mentioned drug.

The agent or pharmaceutical composition for the prophylaxis or treatment of drug-induced neuropathy of the present invention containing CDP-choline as an active ingredient shows a potent improving effect against drug-induced neuropathy and is superior in safety. Consequently, it can be used for the prophylaxis or treatment of drug-induced neuropathy. Moreover, because it shows effect by oral administration, it is also effective for improving QOL (Quality of Life) of patients.

### Examples

The present invention is explained in detail by referring to Examples, which are not to be construed as limitative.

### Example 1

### Test compound: cytidine 5'-diphosphocholine·monosodium salt

(CDP-choline·Na: manufactured by YAMASA CORPORATION) Experiment: Tail Flick Test using vincristine-induced sensory neuropathy mouse (in vivo)

### (1) Preparation of mouse with vincristine-induced sensory neuropathy

Vincristine (0.05 mg/kg for the first time, 0.125 mg/kg for the second time and the following) dissolved in injectable physiological saline was intraperitoneally administered to male ICR strain mice (7-week-old, 10 per group) twice a week for 10 weeks to induce sensory neuropathy.

### (2) Administration of CDP-choline

CDP-choline·Na was dissolved in distilled water, and oral administration thereof (500 mg/kg per dose) was started on the same day as vincristine once a day for 10 weeks.

### (3) Tail Flick Test

A photothermic stimulation was given to the tail of mouse, and the reaction latency up to the motion of the tail upon sensing the heat was measured.

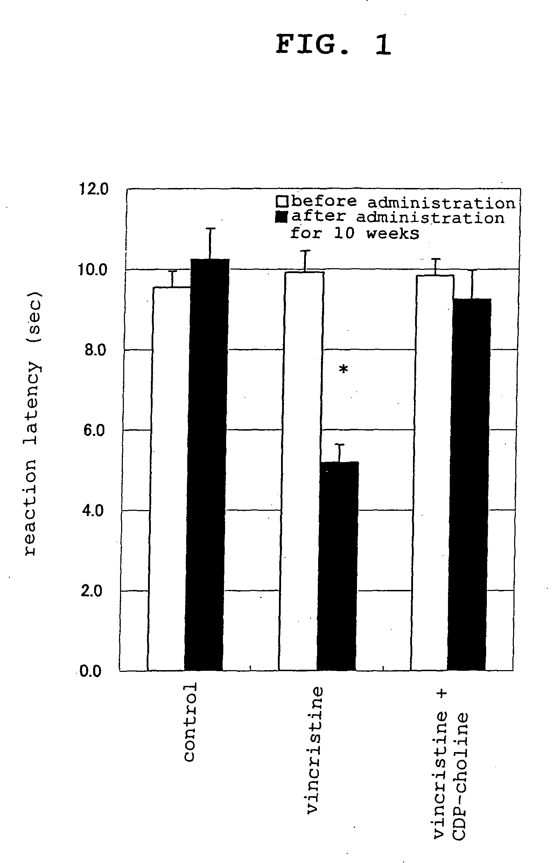

The measurement results of the reaction latency of a control group, a vincristine administration group and a vincristine administration group with administration of CDP-choline (500 mg/kg) are shown in Fig. 1. In the Figure, the measures show mean ± S.E. of the reaction latency of 10 mice, wherein * shows a significant difference (P<0.05) from the control group.

As is clear from Fig. 1, the vincristine administration group showed significantly shortened reaction latency as compared to the control group, thus exhibiting symptom similar to hyperesthesia.

When CDP-choline was administered at 500 mg/kg to deal with the sensory neuropathy observed in this vincristine administration group, the above-mentioned neuropathy was almost completely suppressed and the reaction latency was of the same level as the control group.

From this result, it has been clarified that CDP-choline shows a superior effect on drug-induced neuropathy by oral administration.

### Example 2

### Test compound: cytidine 5'-diphosphocholine·monosodium salt

(CDP-choline·Na: manufactured by YAMASA CORPORATION) Experiment: Tail Flick Test using mice with acrylamide induced sensory neuropathy (in vivo)

### (1) Preparation of mouse with acrylamide-induced sensory neuropathy

Acrylamide (2.5 mg/mice) dissolved in injectable physiological saline was intraperitoneally administered to male ICR strain mice (7-week-old, 10 per group) 5 times a week for 1 week to induce sensory neuropathy.

### (2) Administration of CDP-choline

CDP-choline·Na was dissolved in distilled water, and oral administration thereof (100 mg/kg per dose) was started on the same day as acrylamide once a day for 1 week.

### (3) Tail Flick Test

A photothermic stimulation was given to the tail of mouse, and the reaction latency up to the motion of the tail upon sensing the heat was measured.

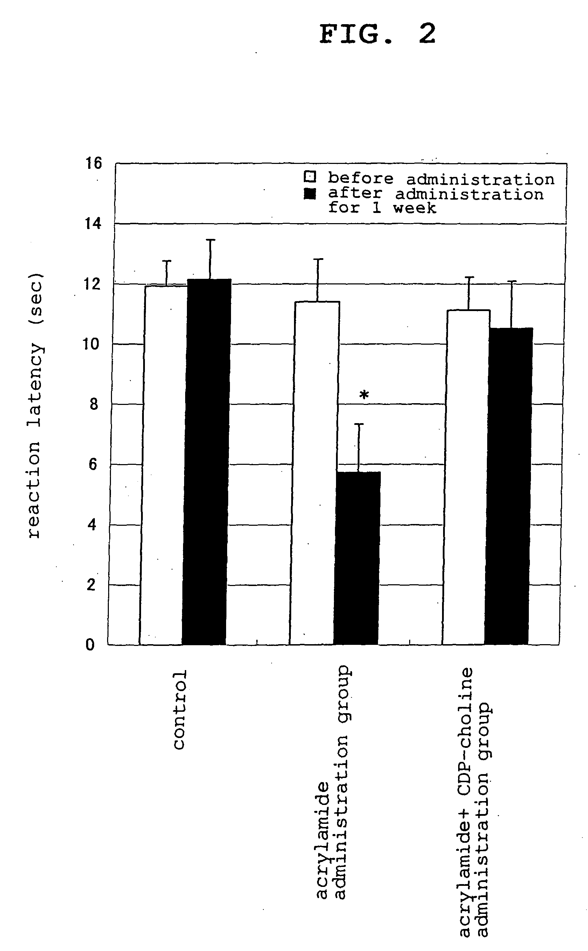

The measurement results of the reaction latency of a control group, an acrylamide administration group and an acrylamide administration group with administration of CDP-choline (100 mg/kg) are shown in Fig. 2. In the Figure, the measures show mean ± S.E. of the reaction latency of 10 mice, wherein * shows a significant difference (P<0.05) from the control group.

As is clear from Fig. 2, the acrylamide administration group showed significantly shortened reaction latency as compared to the control group, thus exhibiting symptom similar to hyperesthesia.

When CDP-choline was administered at 100 mg/kg to deal with the sensory neuropathy observed in this acrylamide administration group, the above-mentioned neuropathy was almost completely suppressed and the reaction latency was of the same level as the control group.

From this result, it has been clarified that CDP-choline shows a superior effect on drug-induced neuropathy by oral administration.

| **Preparation Example 1** tablet | |
|---|---|
| CDP-choline | 30.0 mg |
| microcrystalline cellulose | 25.0 mg |
| lactose | 39.5 mg |
| starch | 40.0 mg |
| talc | 5.0 mg |
| magnesium stearate | 0.5 mg |

Tablets are prepared from the above composition by a conventional method.

| **Preparation Example 2** capsule | |
|---|---|
| CDP-choline | 30.0 mg |
| lactose | 40.0 mg |
| starch | 15.0 mg |
| talc | 5.0 mg |

Capsules are prepared from the above composition by a conventional method.

| **Preparation Example 3** injection | |
|---|---|
| CDP-choline | 30.0 mg |
| glucose | 100.0 mg |

The above composition is dissolved in purified water for injection to prepare injection.

### Industrial Applicability

The agent or pharmaceutical composition for the prophylaxis or treatment of drug-induced neuropathy of the present invention containing CDP-choline as an active ingredient shows a potent improving effect against drug-induced neuropathy and is superior in safety. Consequently, it can be used for the prophylaxis or treatment of drug-induced neuropathy. Moreover, because it shows effect by oral administration, it is also effective for improving QOL (Quality of Life) of patients.

This application is based on a patent application No. 2002-202715 filed in Japan, the contents of which are hereby incorporated by reference.

## Claims

1. An agent for the prophylaxis or treatment of drug-induced neuropathy, which comprises cytidine 5'-diphosphocholine or a pharmaceutically acceptable salt thereof as an active ingredient.

2. The agent of claim 1, wherein the drug-induced neuropathy is peripheral neuropathy.

3. The agent of claim 1, which is in a dosage form for oral administration.

4. The agent of claim 1, wherein the drug-induced neuropathy is induced by a pharmaceutical product selected from the group consisting of antidepressant, antiepileptic agent, antituberculosis agent, antibiotics, anti-malignant tumor agent, a therapeutic agent for AIDS, antirheumatic agent, antiarrhythmic agent, dermatological agent used for Reye syndrome, antialcoholic agent against excessive alcohol drinking, antihypertensive agent, radiation sensitizer, vitamin and immunosuppressant.

5. The agent of claim 1, wherein the drug-induced neuropathy is induced by poisonoum substance or ethanol.

6. A method for the prophylaxis or treatment of drug-induced neuropathy, which comprises administering an effective amount of cytidine 5'-diphosphocholine or a pharmaceutically acceptable salt thereof to a patient.

7. The method of claim 6, wherein the drug-induced neuropathy is peripheral neuropathy.

8. The method of claim 6, wherein the administration comprises oral administration.

9. The method of claim 6, wherein the drug-induced neuropathy is induced by a pharmaceutical product selected from the group consisting of antidepressant, antiepileptic agent, antituberculosis agent, antibiotics, anti-malignant tumor agent, a therapeutic agent for AIDS, antirheumatic agent, antiarrhythmic agent, dermatological agent used for Reye syndrome, antialcoholic agent against excessive alcohol drinking, antihypertensive agent, radiation sensitizer, vitamin and immunosuppressant.

10. The method of claim 6, wherein the drug-induced neuropathy is neuropathy induced by poisonoum substance or ethanol.

11. Use of cytidine 5'-diphosphocholine or a pharmaceutically acceptable salt thereof for the production of an agent for the prophylaxis or treatment of drug-induced neuropathy.

12. Use of claim 11, wherein the drug-induced neuropathy is peripheral neuropathy.

13. Use of claim 11, wherein the agent is in a dosage form for oral administration.

14. Use of claim 11, wherein the drug-induced neuropathy is neuropathy induced by a pharmaceutical product selected from the group consisting of antidepressant, antiepileptic agent, antituberculosis agent, antibiotics, anti-malignant tumor agent, a therapeutic agent for AIDS, antirheumatic agent, antiarrhythmic agent, dermatological agent used for Reye syndrome, antialcoholic agent against excessive alcohol drinking, antihypertensive agent, radiation sensitizer, vitamin and immunosuppressant.

15. Use of claim 11, wherein the drug-induced neuropathy is neuropathy induced by a poisonoum substance or ethanol.

16. A pharmaceutical composition for the prophylaxis or treatment of drug-induced neuropathy, which comprises an effective amount of cytidine 5'-diphosphocholine or a pharmaceutically acceptable salt thereof, and a pharmaceutically acceptable carrier.

17. The pharmaceutical composition of claim 16, wherein the drug-induced neuropathy is peripheral neuropathy.

18. The pharmaceutical composition of claim 16, which is in a dosage form for oral administration.

19. The pharmaceutical composition of claim 16, wherein the drug-induced neuropathy is neuropathy induced by a pharmaceutical product selected from the group consisting of antidepressant, antiepileptic agent, antituberculosis agent, antibiotics, anti-malignant tumor agent, a therapeutic agent for AIDS, antirheumatic agent, antiarrhythmic agent, dermatological agent used for Reye syndrome, antialcoholic agent against excessive alcohol drinking, antihypertensive agent, radiation sensitizer, vitamin and immunosuppressant.

20. The pharmaceutical composition of claim 16, wherein the drug-induced neuropathy is neuropathy induced by a poisonoum substance or ethanol.

21. A commercial package comprising the pharmaceutical composition of claim 16 and a written matter associated therewith, the written matter stating that the pharmaceutical composition can or should be used for the prophylaxis or treatment of drug-induced neuropathy.
